# EUROPÄISCHE PATENTANMELDUNG

(11) **EP 4 649 972 A1**
(43) Veröffentlichungstag der Anmeldung: **19.11.2025**
(21) Anmeldenummer: 25171410.1
(22) Anmeldetag: 17.04.2025
(51) Int. Cl.: A61M 5/00, A61M 5/162

(54) **INFUSIONSSYSTEM**

(30) Priorität: 13.05.2024 DE 202024000937 U
(71) Anmelder: Senn, Christine, 80992 München (DE); Sulejmani, Asra, 81249 München (DE)
(72) Erfinder: Senn, Christine, 80992 München (DE); Sulejmani, Asra, 81249 München (DE)
(74) Vertreter: Berghofer, Benedikt

(57) **Zusammenfassung**

Ein erfindungsgemäßes Infusionssystem (30) umfasst ein Infusionsbesteck (2) und eine Verpackung (32), in der das Infusionsbesteck (2) steril verpackt ist und mit einer Infusionsflüssigkeit (14) vorbefüllt ist.

## Beschreibung

Die vorliegende Erfindung betrifft ein Infusionssystem umfassend ein Infusionsbesteck und eine Verpackung, in der das Infusionsbesteck steril verpackt ist.

Ein Infusionsbesteck wird zur Verbindung eines Infusionsbehälters, der eine Infusionsflüssigkeit enthält, mit einem Zugang, beispielsweise einem intravenösen Zugang, verwendet, um einem Patienten die Infusionsflüssigkeit zu verabreichen. Vor der Verabreichung der Infusion muss das Infusionsbesteck manuell mit dem Infusionsbehälter verbunden, mit der Infusionsflüssigkeit befüllt und entlüftet werden. Dieser Vorgang ist sehr mühsam und zeitintensiv, insbesondere wenn eine Vielzahl von Infusionen vorzubereiten ist, wie zum Beispiel in einem Krankenhaus. Da Antibiosen und andere medikamentöse Behandlungen maximal eine Stunde vor Verabreichung vorbereitet werden dürfen und der Bedarf an Infusionen beispielsweise in einem Krankenhaus sehr hoch ist, muss eine Vielzahl von Infusionen in kurzer Zeit vorbereitet werden. Oftmals wird daher zu Schichtbeginn zunächst eine große Anzahl von Infusionen vorbereitet. Dabei darf die Vorbereitung nur durch medizinisches Fachpersonal erfolgen, an dem es bekanntermaßen ohnehin mangelt und das in Folge dieser Vorbereitungsarbeiten weniger Zeit zur Betreuung von Patienten zur Verfügung hat.

Im Allgemeinen und auch im Rahmen der vorliegenden Erfindung umfasst das Vorbereiten des Infusionsbestecks bzw. der Infusion die folgenden Schritte:
- Entnehmen des Infusionsbestecks aus der Verpackung und Schließen eines Durchflussreglers des Infusionsbestecks;
- Entfernen einer Deckfolie oder Schutzkappe vom Infusionsbehälter und gegebenenfalls desinfizieren des den Infusionsbehälter verschließenden Gummiseptums/Ports;
- Einstechen eines Einstechdorns des Infusionsbestecks in den stehenden Infusionsbehälter;
- Umdrehen des Infusionsbehälters und Pumpen der Infusionsflüssigkeit in eine Tropfkammer des Infusionsbestecks;
- Öffnen des Durchflussreglers und Füllen des gesamten Infusionsschlauchs bis zu einem Anschlusskonnektor;
- Prüfen, dass patientenseitig keine Luftblasen vorhanden sind, und gegebenenfalls Entlüften des Systems, bis alle Luftblasen entfernt sind, wobei der Durchflussregler üblicherweise mehrmals zu öffnen und zu schließen ist.

In der Praxis stehen dabei häufig weniger Aufhängevorrichtungen zur Verfügung, als Infusionen vorzubereiten sind. Die Infusionsbehälter samt Infusionsbesteck sind daher manuell hochzuhalten, etwa auf Kopfhöhe, damit das Infusionsbesteck befüllt und entlüftet werden kann. Dies ist bei der erforderlichen Stückzahl mit großer körperlicher Anstrengung verbunden. Auch das häufige Öffnen und Schließen des Durchflussreglers hinterlässt Spuren an den Händen des medizinischen Personals.

Trotz der Anstrengung und des zeitlichen Drucks erfordert das Vorbereiten des Infusionsbestecks bzw. der Infusionen ein überaus hohes Maß an Sorgfalt, da im System verbleibende Luftblasen eine Gefährdung der Patienten bedeuten. Beispielsweise könnte es zu Embolien, Thrombosen, etc. kommen. Der zeitliche Druck steigt noch weiter, wenn es sich um akute Situationen handelt, insbesondere in lebensbedrohlichen Situationen, in denen z.B. die Gabe von Infusionen zur Schmerzlinderung, Stabilisierung des Kreislaufs oder eine akute medikamentöse Behandlung erforderlich ist.

Es ist daher eine Aufgabe der vorliegenden Erfindung, ein Infusionssystem bereitzustellen, das eine einfache, schnelle und zugleich zuverlässige Vorbereitung des Infusionsbestecks bzw. der Infusion ermöglicht.

Diese Aufgabe wird durch den Gegenstand des Anspruchs 1 gelöst. Bevorzugte Ausführungsformen sind Gegenstand der abhängigen Ansprüche.

Ein erfindungsgemäßes Infusionssystem umfasst ein Infusionsbesteck und eine Verpackung, in der das Infusionsbesteck steril verpackt ist, und zeichnet sich dadurch aus, dass das Infusionsbesteck in der Verpackung mit einer Infusionsflüssigkeit vorbefüllt ist.

Auf diese Art und Weise wird das medizinische Fachpersonal körperlich und zeitlich erheblich entlastet und menschliche Fehler bei der Vorbereitung von Infusionen können reduziert werden. Das manuelle Befüllen und das Entlüften des Infusionsbestecks können auf ein Minimum reduziert werden, da das Infusionsbesteck im Wesentlichen fertig befüllt und entlüftet in der Verpackung bereitgestellt wird. Es bedarf lediglich der Verbindung mit dem Infusionsbehälter und einer finalen Kontrolle, wodurch die für die Vorbereitung erforderliche Zeit erheblich verkürzt werden kann. Im Schichtbetrieb eines Krankenhauses kann dies eine Zeitersparnis von 1 bis 2 Stunden pro Fachkraft, die mit dem Vorbereiten der Infusionen betraut ist, pro Schicht bedeuten. Dem medizinischen Fachpersonal bleibt somit deutlich mehr Zeit für die Patientenbetreuung. Zugleich wird die Sicherheit für die Patienten erhöht, da das Infusionsbesteckt bereits im Wesentlichen entlüftet ist und somit das Risiko von Folgeerscheinungen durch noch im System befindliche Luftblasen, wie z.B. von Thrombosen oder Embolien, reduziert ist. Schließlich wird durch die Reduzierung der manuellen Vorbereitungsschritte nach dem Entnehmen des Infusionsbestecks aus der sterilen Verpackung auch das Kontaminationsrisiko reduziert.

Bei dem Infusionssystem handelt es sich insbesondere um ein Infusionssystem für medizinische Zwecke, z.B. zur Infusion von Kochsalzlösung oder medikamentösen Lösungen, wie Schmerzmittel oder Antibiotika. Das Infusionsbesteck, das auch als Infusionsgerät bezeichnet wird, kann z.B. ein Transfusionsbesteck, ein Perfusor-Infusionsbesteck, ein Kinder-Infusionsbesteck oder dergleichen sein. Besonders bevorzugt ist das Infusionsbesteck ein Schwerkraft-Infusionsbesteck.

Vorzugsweise umfasst das Infusionsbesteck einen Einstechdorn zum Einstechen in einen Infusionsbehälter, eine Tropfkammer in Fluidverbindung mit dem Einstechdorn, einen Infusionsschlauch in Fluidverbindung mit der Tropfkammer und einen Durchflussregler zum Regeln des Durchflusses der Infusionsflüssigkeit durch den Infusionsschlauch. Der Einstechdorn kann direkt an die Tropfkammer anschließen, ggf. aber auch über einen Rohr- oder Schlauchabschnitt mit dieser verbunden sein. Eine Belüftungsklappe kann an dem Einstechdorn oder an der Tropfkammer vorgesehen sein. Die Belüftungsklappe ist vorzugsweise geschlossen, wenn das Infusionsbesteck in der Verpackung aufgenommen ist. Dadurch wird der Eintritt von Luftblasen in den Infusionsschlauch unterbunden. Der Infusionsschlauch ist vorzugsweise an einem Ende mit der Tropfkammer verbunden und am anderen Ende mit einem Anschlusskonnektor, z.B. einem Luer Lock Adapter, verbunden. Der Infusionsschlauch kann einteilig oder mehrteilig ausgebildet sein. Beispielsweise kann es sich bei dem Anschlusskonnektor auch um einen Drei-Wege-Hahn handeln, der wiederum mittels eines Verbindungsschlauchs mit einem weiteren Anschlusskonnektor verbunden ist. Der Durchflussregler ist vorzugsweise als Rollklemme ausgebildet. Optional kann das Infusionsbesteck weiterhin einen Infusionsfilter aufweisen, der mit dem Infusionsschlauch derart in Fluidverbindung steht, dass die Infusionsflüssigkeit durch den Infusionsfilter hindurch zum Anschlusskonnektor fließt. Bei dem Infusionsbehälter handelt es sich üblicherweise um einen Beutel oder eine Flasche, wobei letztere aus Kunststoff oder Glas gebildet sein kann.

Die Tropfkammer und der Infusionsschlauch sind vorzugsweise mit Infusionsflüssigkeit vorbefüllt. Bevorzugt ist die Tropfkammer zumindest teilweise und der Infusionsschlauch vollständig mit der Infusionsflüssigkeit vorbefüllt. Dabei ist die Tropfkammer vorzugweise bis zu einem vorbestimmten Füllstand, der beispielsweise herstellerseitig vorgegeben sein kann, vorbefüllt. Der Infusionsschlauch ist vorzugweise vollständig vorbefüllt, insbesondere einschließlich des Anschlusskonnektors bzw. allen von der Tropfkammer aus gesehen patientenseitigen Komponenten des Infusionsbestecks. Außerdem ist bevorzugt zumindest der Infusionsschlauch entlüftet, enthält also keine Luftblasen mehr. Dies trifft vorzugsweise auf alle von der Tropfkammer aus gesehen patientenseitigen Komponenten des Infusionsbestecks zu.

Die Menge von Infusionsflüssigkeit, mit der das Infusionsbesteck vorbefüllt ist, ist abhängig von der Ausführungsform und dem Einsatzbereich und der dadurch bedingten Größe des Infusionsbestecks, insbesondere vom Volumen des Infusionsschlauchs. Beispielsweise können Infusionsbestecke zum Einsatz bei Kleinkindern und Kindern kleiner dimensioniert sein als solche zum Einsatz bei Erwachsenen. Bevorzugt ist das Infusionsbesteck mit 3 ml bis 20 ml, mehr bevorzugt mit 5 ml bis 15 ml Infusionsflüssigkeit vorbefüllt. Kleinere Infusionsbestecke, beispielsweise für (Klein-) Kinder, sind vorzugsweise mit ca. 5 ml Infusionsflüssigkeit vorbefüllt. Größere Infusionsbestecke, beispielsweise für Erwachsene, sind vorzugsweise mit 9 ml bis 13 ml Infusionsflüssigkeit vorbefüllt.

Vorzugsweise ist die Infusionsflüssigkeit eine Infusionslösung, insbesondere eine Kochsalzlösung oder Wasser für Injektionszwecke. Bei der beabsichtigten Verabreichung medikamentöser Lösungen, wie z.B. bei Schmerzmittelinfusionen oder Antibiotikainfusionen, ist in der Regel eine vorherige Spülung des Infusionsbestecks mit Kochsalzlösung oder Wasser für Injektionszwecke erforderlich. Bei Kochsalzlösung und Wasser für Injektionszwecke handelt es sich daher um sehr häufig verabreichte Infusionsflüssigkeiten, weshalb sich ihre Verwendung hier anbietet. Wasser für Injektionszwecke wird auch als Aqua, genauer als Aqua ad iniectabile oder Aqua ad injectionem bezeichnet. Bei der Kochsalzlösung handelt es sich vorzugsweise um eine 0,9 %-ige Kochsalzlösung, also eine Lösung, die ca. 9 g Kochsalz pro Liter aufweist.

Bevorzugt ist das Infusionsbesteck verschlossen, insbesondere luftdicht und/oder flüssigkeitsdicht verschlossen. So kann das Infusionsbesteck vorbefüllt und verschlossen in der Verpackung aufgenommen sein, ohne dass Infusionsflüssigkeit austritt oder Luft in das System eintritt. Vorzugsweise ist das Infusionsbesteck am Einstechdorn, beispielsweise mittels einer ersten Kappe, und/oder am Infusionsschlauch oder Anschlusskonnektor, beispielsweise mittels einer zweiten Kappe, verschlossen. Die erste und/oder die zweite Kappe sind vorzugsweise aus Kunststoff, beispielsweise aus Gummi oder Silikon, gebildet. Es wäre auch denkbar, eine Membran, insbesondere eine Burst- oder Durchstechmembran, zum Verschließen des Infusionsbestecks zu verwenden.

Die Verpackung ist vorzugweise geschlossen und sterilisiert, sodass das Infusionsbesteck in der Verpack steril aufgenommen ist. In diesem Zustand ist das Infusionsbesteck bereits mit der Infusionsflüssigkeit vorbefüllt. Dadurch bleiben dem medizinischen Fachpersonal manuelle Vorbereitungsschritte erspart, was zu einer zeitlichen und körperlichen Entlastung und einem verminderten Kontaminationsrisiko führt.

Bei der Verpackung handelt es sich vorzugsweise um eine Peel-Verpackung, einen versiegelten Beutel oder einen versiegelten Schlauch. Besonders bevorzugt ist die Verpackung eine Verpackung für in der Endverpackung zu sterilisierende Medizinprodukte nach DIN EN 868-5 (2019) und/oder DIN EN ISO 11607-1 (2020). Eine Peel-Verpackung umfasst vorzugsweise ein Trägermaterial und eine an das Trägermaterial gesiegelte oder geklebte Klarsichtfolie. Diese Art von Verpackungen hat sich im Bereich der Medizinprodukte bewährt.

Das Infusionssystem gemäß der vorliegenden Erfindung ist in allen medizinischen Bereichen anwendbar, wie zum Beispiel in Kinderkliniken, Erwachsenenkliniken, Normalstationen, Intensivstationen, Kinderintensivstationen, im Bereich der Onkologie, in Rehabilitationskliniken, in Arztpraxen, Gesundheitszentren, Praxiskliniken oder im Rettungsdienst.

Weitere Merkmale und Vorteile der vorliegenden Erfindung ergeben sich aus der nachfolgenden Beschreibung unter Bezugnahme auf die beigefügten Zeichnungen.
- Fig. 1: zeigt die Komponenten eines Infusionsbestecks.
- Fig. 2: zeigt ein erfindungsgemäßes Infusionssystem mit einem Infusionsbesteck nach Fig. 1 und einer Verpackung, in der das Infusionsbesteck aufgenommen ist.

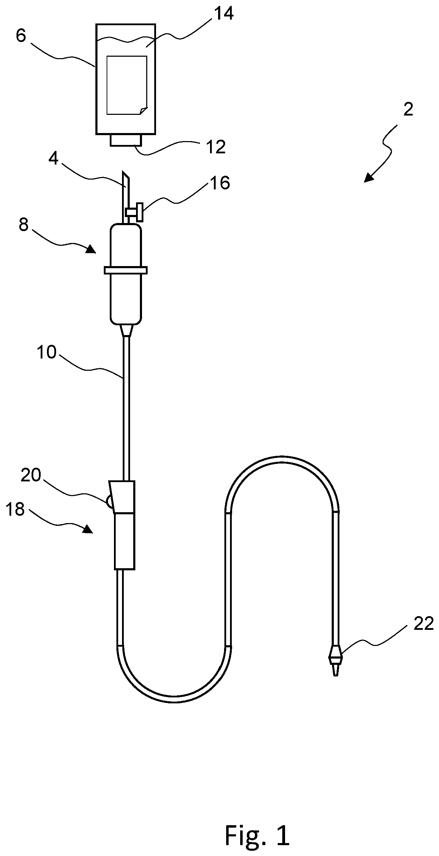

In Fig. 1 ist ein Infusionsbesteck 2 schematisch dargestellt. Das Infusionsbesteck 2 umfasst in der dargestellten Ausführungsform einen Einstechdorn 4 zum Einstechen in einen Infusionsbehälter 6, eine Tropfkammer 8 in Fluidverbindung mit dem Einstechdorn 4 und einen Infusionsschlauch 10 in Fluidverbindung mit der Tropfkammer 8. Der Einstechdorn 4 schließt hier direkt an die Tropfkammer 8 an.

Der Infusionsbehälter 6 kann z.B. ein Beutel oder eine Flasche sein. Der Infusionsbehälter 6 kann eine Deckfolie oder Schutzkappe aufweisen, nach deren Entfernung ein Port 12 des Infusionsbehälters 6 freiliegt. Der Port 12 kann eine Membran oder ein Gummiseptum aufweisen, durch die der Einstechdorn 4 hindurchgestochen wird, um eine Fluidverbindung zwischen dem Infusionsbehälter 6 und der Tropfkammer 8 herzustellen. Der Infusionsbehälter 6 enthält eine Infusionsflüssigkeit 14, die dann durch den Einstechdorn 4 in das Infusionsbesteck 2 strömen kann. Eine Belüftungsklappe 16 kann am Einstechdorn 4, einem Verbindungselement zwischen dem Einstechdorn 4 und der Tropfkammer 8 oder der Tropfkammer 8 selbst vorgesehen sein und ist zu öffnen, um ein durch die Schwerkraft bedingtes Strömen von Infusionsflüssigkeit 14 in das Infusionsbesteck 2 zu ermöglichen.

Vorzugsweise umfasst das Infusionsbesteck 2 weiterhin einen Durchflussregler 18, hier in Form einer Rollklemme, zum Regulieren des Durchflusses von Infusionsflüssigkeit 14 durch den Infusionsschlauch 10. Bei herkömmlichen Infusionsbestecken ist die Rollklemme zum Entlüften des Infusionsschlauchs 10 mittels der Rolle 20 zu öffnen und zu schließen, was vor allem bei einer großen Anzahl vorzubereitender Infusionen sehr mühsam für das Personal ist.

Am Ende des Infusionsschlauchs 10 befindet sich bevorzugt ein Anschlusskonnektor 22, z.B. in Form eines Luer Lock Adapters. Der Anschlusskonnektor 22 kann entweder direkt an eine Kanüle angeschlossen werden oder mit einem Drei-Wege-Hahn verbunden werden, an dem wiederum ein Verbindungsschlauch mit einem weiteren Anschlusskonnektor zum Anschluss an die Kanüle vorgesehen ist.

Grundsätzlich sind dem Fachmann verschiedene Ausführungsformen von Infusionsbestecken 2 bekannt, die mittels der eingangs beschriebenen Schritte vorzubereiten und mit einem Infusionsbehälter 6 zu verbinden wären. Damit ist ein großer personeller und zeitlicher Aufwand verbunden, der insbesondere bei Personalmangel oder in zeitkritischen Situationen problematisch ist und ein gewisses Sicherheitsrisiko darstellt.

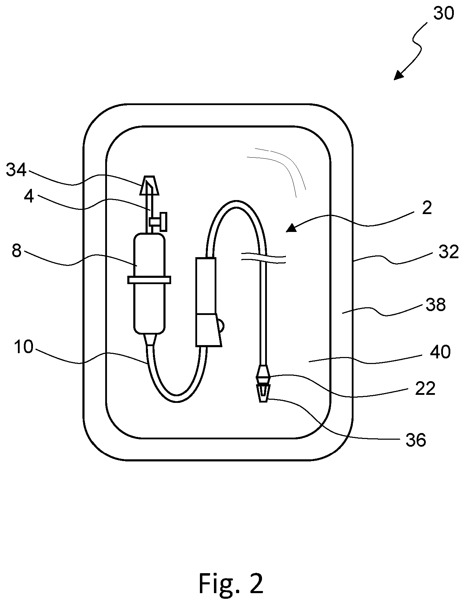

Ein erfindungsgemäßes Infusionssystem 30 ist in Fig. 2 dargestellt. Das Infusionssystem 30 umfasst das Infusionsbesteck 2 und eine Verpackung 32, in der das Infusionsbesteck 2 steril verpackt ist und bereits mit einer Infusionsflüssigkeit 14 vorbefüllt ist. Dabei sind bevorzugt zumindest die Tropfkammer 8 bis zu einem vorbestimmten Füllstand sowie der Infusionsschlauch 10 und der Anschlusskonnektor 22 vollständig mit Infusionsflüssigkeit 14 vorbefüllt. Dadurch kann das Befüllen und Entlüften des Infusionsbestecks 2 bereits herstellerseitig erfolgen, wodurch das medizinische Fachpersonal erheblich entlastet wird und das Risiko menschlicher Fehler bei der Vorbereitung minimiert wird. Es ist lediglich die Verpackung 32 zu öffnen und das Infusionsbesteck 2 mit einem Infusionsbehälter 6 zu verbinden.

Damit im verpackten und vorbefüllten Zustand keine Infusionsflüssigkeit 14 austreten kann, ist das Infusionsbesteck 2 vorzugsweise verschlossen, beispielsweise mittels einer ersten Kappe 34 am Einstechdorn 4 und einer zweiten Kappe 36 am Anschlusskonnektor 22. Es kann auch ein einseitiges verschließen ausreichen, insbesondere wenn die Belüftungsklappe 16 geschlossen ist.

Bei der Verpackung 32 kann es sich um eine Peel-Verpackung handeln, die ein Trägermaterial 38 und eine Klarsichtfolie 40 umfasst, die umlaufend an das Trägermaterial 38 gesiegelt oder geklebt ist. Zwischen dem Trägermaterial 38 und der Klarsichtfolie 40 ist ein Hohlraum zur Aufnahme des Infusionsbestecks 2 ausgebildet. Derartige Verpackungen haben sich zur Aufnahme von zu sterilisierenden Medizinprodukten als besonders geeignet erwiesen.

Verschiedene Abwandlungen der durch die beigefügten Ansprüche definierten Erfindung sind dem Fachmann basierend auf der hierin enthaltenen, detaillierten Beschreibung bevorzugter Ausführungsformen ersichtlich.

## Patentansprüche

1. Infusionssystem (30) umfassend ein Infusionsbesteck (2) und eine Verpackung (32), in der das Infusionsbesteck (2) steril verpackt ist, **dadurch gekennzeichnet, dass** das Infusionsbesteck (2) in der Verpackung (32) mit einer Infusionsflüssigkeit (14) vorbefüllt ist.

2. Infusionssystem (30) nach Anspruch 1, **dadurch gekennzeichnet, dass** die Infusionsflüssigkeit (14) eine Infusionslösung, insbesondere eine Kochsalzlösung oder Wasser für Injektionszwecke ist.

3. Infusionssystem (30) nach Anspruch 1 oder 2, **dadurch gekennzeichnet, dass** das Infusionsbesteck (2) umfasst: einen Einstechdorn (4) zum Einstechen in einen Infusionsbehälter (6), eine Tropfkammer (8) in Fluidverbindung mit dem Einstechdorn (4), einen Infusionsschlauch (10) in Fluidverbindung mit der Tropfkammer (8) und einen Durchflussregler (18) zum Regeln des Durchflusses durch den Infusionsschlauch (10), wobei zumindest die Tropfkammer (8) und der Infusionsschlauch (10) mit der Infusionsflüssigkeit (14) vorbefüllt sind.

4. Infusionssystem (30) nach Anspruch 3, **dadurch gekennzeichnet, dass** der Infusionsschlauch (10) vollständig und die Tropfkammer (8) zumindest teilweise mit der Infusionsflüssigkeit (14) vorbefüllt sind.

5. Infusionssystem (30) nach Anspruch 3 oder 4, **dadurch gekennzeichnet, dass** das Infusionsbesteck (2) verschlossen ist.

6. Infusionssystem (30) nach Anspruch 5, **dadurch gekennzeichnet, dass** das Infusionsbesteck (2) mittels einer ersten Kappe (34) am Einstechdorn (4) und/oder einer zweiten Kappe (36) am Infusionsschlauch (10), insbesondere an einem Anschlusskonnektor (22) des Infusionsschlauchs (10), verschlossen ist.

7. Infusionssystem (30) nach einem der Ansprüche 3 bis 6, **dadurch gekennzeichnet, dass** das Infusionsbesteck (2) eine Belüftungsklappe (16) aufweist, die geschlossen ist.

8. Infusionssystem (30) nach einem der vorangehenden Ansprüche, **dadurch gekennzeichnet, dass** das Infusionsbesteck (2) vorbefüllt ist mit 3 ml bis 20 ml, vorzugsweise mit 5 ml bis 15 ml, mehr bevorzugt mit 9 ml bis 13 ml der Infusionsflüssigkeit (14).

9. Infusionssystem (30) nach einem der vorangehenden Ansprüche, **dadurch gekennzeichnet, dass** die Verpackung (32) eine Peel-Verpackung, ein versiegelter Beutel oder ein versiegelter Schlauch ist.
